# NEUE EUROPÄISCHE PATENTSCHRIFT

(11) **EP 3 000 420 B2**
(45) Veröffentlichungstag und Bekanntmachung des Hinweises auf die Entscheidung über den Einspruch: **26.06.2024**
(45) Hinweis auf die Patenterteilung: 12.10.2016
(21) Anmeldenummer: 15020170.5
(22) Anmeldetag: 23.09.2015
(51) Int. Cl.: A61B 17/22

(54) **GERÄT ZUR BEHANDLUNG DES MENSCHLICHEN ODER TIERISCHEN KÖRPERS MIT MECHANISCHEN STÖSSEN**
DEVICE FOR TREATMENT OF THE HUMAN OR ANIMAL BODY WITH MECHANICAL IMPACTS
APPAREIL DE TRAITEMENT DU CORPS HUMAIN OU ANIMAL A L'AIDE DE CHOCS MECANIQUES

(30) Priorität: 26.09.2014 DE 202014007694 U
(43) Veröffentlichungstag der Anmeldung: 30.03.2016
(73) Patentinhaber: Storz Medical AG, 8274 Tägerwilen (CH)
(72) Erfinder: Novak, Pavel, 8234 Stetten (CH); Schulz, Johannes Manfred, 8274 Tägerwilen (CH); Hagelauer, Ulrich, 78464 Konstanz (DE); Görner, Georg, 8597 Landschlacht/TG (CH)
(74) Vertreter: König Szynka Tilmann von Renesse Patentanwälte Partnerschaft mbB München

(56) Entgegenhaltungen:
- DE-A1- 2 735 563
- DE-A1- 3 048 293
- DE-A1-102004 042 895
- DE-C1- 19 624 446
- DE-U1-202010 009 890
- US-A- 4 298 074
- US-A- 5 735 855
- US-A1- 2010 256 536
- US-A1- 2011 054 367
- US-A1- 2012 029 393
- US-B1- 6 213 971

## Beschreibung

Die vorliegende Erfindung bezieht sich auf ein Gerät zur Behandlung des menschlichen oder tierischen Körpers durch Ausüben von Stößen auf die Körperoberfläche. Im Folgenden wird zur Vereinfachung vom Körper eines Patienten gesprochen, der bevorzugt menschlich ist.

Im Stand der Technik sind verschiedene Geräte des beschriebenen Grundtyps bekannt. Die DE 197 25 477 C beschreibt bspw. ein solches Gerät, bei dem durch die Kollision eines pneumatisch beschleunigten Schlagteils oder Projektils mit einem zunächst ruhenden Prallkörper oder Applikator eine Druckwelle ausgelöst wird, die dadurch in den Körper des Patienten eingekoppelt werden kann, dass eine Applikatorfrontfläche zum Zeitpunkt der Kollision auf den Patientenkörper aufgelegt wird. Dieser Gerätetyp leitet sich in seiner Entstehungsgeschichte von Lithotripsiegeräten ab, bei denen solche Druckwellen z. B. über eine lange stabförmige Sonde an der Frontfläche des Applikators auf einen Nierenstein oder Ähnliches übertragen werden können, um diesen zu desintegrieren.

Der Schwerpunkt bei der Darstellung liegt in jedem Fall auf der durch die Kollision erzeugten Druckwelle, die mehr oder weniger einer eigentlichen Stoßwelle ähneln soll, wie sie klassische Lithotripsiegeräte z. B. mit piezoelektrischen oder induktiven Aktuatoren und Fokussierung auf einen Stein erzeugen. Solche Druckwellen können Anstiegsflanken mit einer Breite im Bereich weniger µs und einer Amplitude im niedrigen zweistelligen MPa-Bereich haben (z. B. 2 µs und 15 MPa gemessen 1 cm vor der Frontfläche). In dem zitierten Dokument wird hingegen betont, dass die physikalisch an sich unvermeidliche makroskopische Schwerpunktbewegung des Applikators möglichst klein gehalten werden soll, weil sie als störend angesehen wird.

Als zweites Beispiel wird auf die DE 20 2004 011 323 U und, mit sehr ähnlichem Inhalt, die US 2011/0054367 A1 verwiesen. Dort wird ein hinsichtlich des technischen Aufbaus ähnliches Gerät beschrieben, bei dem allerdings u. a. die elastische Aufhängung des Applikators im Gehäuse auf größere Schwerpunktsbewegungen des Applikators ("Hübe") ausgelegt ist. Dort wird betont, dass die therapeutische Wirkung durchaus auch oder vorwiegend in den eigentlichen makroskopischen Stößen (also infolge des Hubes) gesehen werden kann, was auch von der Indikation abhängt.

Zum Stand der Technik wird ferner verwiesen auf die US 2010/0256536 A1, bei der ein einschlägiger Applikator mit einer abschraubbaren Applikatorkappe gehalten ist. Die zweiteilige Form des Anspruchs 1 basiert auf diesem Dokument.

Ferner ist zu verweisen auf die US 4,298,074, die US 5,435,855 und schließlich die US 6,213,971 B1, die alle verschiedene chirurgische Geräte zeigen, bei denen ein Werkzeug in einer außerhalb des Gerätegehäuses vorgesehenen Kupplung auswechselbar gehalten ist.

Die vorliegende Erfindung richtet sich allgemein auf Geräte des eingangs beschriebenen Bautyps, und zwar sowohl hinsichtlich der Anwendung von Druckwellen als auch hinsichtlich der Anwendung "makroskopischer Stöße" des Applikator auf die Körperoberfläche.

Dabei liegt der Erfindung die Aufgabe zugrunde, ein solches Gerät hinsichtlich weitergehender Anwendungsmöglichkeiten weiterzubilden.

Die Aufgabe wird gelöst durch ein Gerät nach Anspruch 1 und eines nach Anspruch 2. Erfindungsgemäß ist dabei der Applikator in einer lösbaren Kupplung gehalten, so dass zumindest ein distales Applikatorteil aus dem Gerät nach vorn herausgezogen werden kann. Zur Klarstellung: Die Begriffe "proximal" und "distal" werden hier aus der Perspektive eines Benutzers des Geräts gebraucht; in anderen Worten bedeutet "distal" eine dem Patienten zugewandte Position und "proximal" eine dazu entgegengesetzte. Im gleichen Sinn wird als "vorn" die distale Richtung bezeichnet, also auf den Patienten zu.

Die Erfinder haben festgestellt, dass die Optimierung der Geräteeigenschaften im Hinblick auf bestimmte Behandlungen zu einem wesentlichen Teil den sogenannten Applikator betrifft, also zunächst das Teil, das auf den Patienten aufgesetzt wird. Hierbei können zwar noch zwischengeschaltete Elemente auftauchen, z. B. aus Hygienegründen verwendete Überzüge oder Ankopplungsmedien wie Vaseline oder Ultraschallgel; im Wesentlichen geht es jedoch um ein Aufsetzen des Applikators auf den Patienten.

Außerdem ist der Applikator in dem Gerät selbst zur Stoßerzeugung vorgesehen, z. B. bei einer bevorzugten Ausgestaltung durch Aufprall eines beschleunigten Projektils auf eine Applikatorfläche. Das Projektil kann dabei z. B. elektrodynamisch oder anderweitig beschleunigt werden, vorzugsweise pneumatisch. Ferner sind auch andere Stoßerzeugungsmechanismen denkbar, z. B. durch Beaufschlagen des Applikators mit einem Druckgaspuls an sich und ohne Projektil.

Ein Applikator in diesem Sinn kann mehrteilig aufgebaut sein, wobei die Entnehmbarkeit gemäß dieser Erfindung primär ein distales Teil betrifft. Dieses distale Teil des Applikators kann dann gegen andere entsprechende distale Applikatorteile getauscht werden, z. B. weil das Applikatorteil nur eine begrenzte Lebensdauer hat, weil es gereinigt oder sterilisiert werden soll oder weil ein anderer Typ dieses distalen Applikatorteils (oder des gesamten Applikators) verwendet werden soll. Insbesondere ist es möglich, in ihrem distalen Teil unterschiedlich geformte Applikatoren, möglicherweise auch aus verschiedenen Materialien, einzusetzen.

Der Austausch erfolgt dabei sehr einfach und schnell, vorzugsweise völlig werkzeuglos und erfindungsgemäß jedenfalls ohne Abbauen eines weiteren Geräteteils, etwa einer Aufschraubkappe bei konventionellen Geräten dieses Typs. Bei diesen konventionellen Geräten war der Applikator in der Regel einteilig und innerhalb des Gerätegehäuses etwas dicker (senkrecht zur Stoßrichtung) als eine vordere Öffnung. Ein Ausbau war daher möglich, aber nur nach Teilzerlegung des Gehäuses, nämlich Abschrauben einer Schraubkappe und nicht durch Lösen einer für den Applikatorwechsel vorgesehenen Kupplung.

Eine bevorzugte Ausführungsform sieht eine verdrehsichere Montage des Applikators in dem Gerät vor (nicht zwingend in der Kupplung). Es können nämlich Applikatorformen gewünscht sein, die bezüglich der Stoßrichtung nicht rotationssymmetrisch sind, wobei womöglich genau solche Abweichungen wichtig sind. Dementsprechend kann z. B. die Kupplung selbst eine Verdrehsicherheit herstellen oder durch Einschieben eines entsprechend gestalteten Bereichs des Applikators in eine entsprechende Aufnahme eine Verdrehsicherheit hergestellt werden, besonders bevorzugterweise durch einen Mehrkantstift auf der Applikatorseite und eine dazu passende Aufnahme auf der Geräteseite, etwa durch einen hexagonalen Formschluss. Diese Verdrehsicherung betrifft dabei bevorzugt zumindest auch eine von der (die Bewegung in Stoßrichtung betreffenden) Kupplung selbst unabhängige Aufnahme, um die Möglichkeiten zur Kraftaufnahme zu verbessern. Zur Veranschaulichung wird auf das Ausführungsbeispiel verwiesen.

Die Kupplung kann gemäß Anspruch 1 ebenfalls mit einem Formschluss arbeiten, der nicht zwingend mit dem Formschluss für die Verdrehsicherung übereinstimmen muss. Insbesondere ist dann eine Klemmhülse vorgesehen, die (vorzugsweise in Stoßrichtung) verschiebbar ist und dabei zumindest ein Formschlusselement in eine entsprechende Aufnahme an dem Applikator hereindrückt bzw. beim Lösen freigibt. Insbesondere kann ein Gehäuseteil des Geräts gegen eine Federkraft verschiebbar sein und dabei die Klemmhülse aufweisen oder mitverschieben. Als Formschlusselement kommt eine Kugel in Betracht, wobei vorzugsweise mindestens zwei Kugeln vorgesehen sind. Die Kugeln oder andere Formschlusselemente können durch eine schräge Fläche an einer Innenseite der Klemmhülse beaufschlagt werden.

Wie bereits erwähnt wurde, muss der Applikator nicht zwingend einteilig sein. Beim Ausführungsbeispiel ist ein distales Applikatorteil entnehmbar durch Lösen der Kupplung und ein proximales Applikatorteil bleibt dabei in dem Gerätegehäuse. Dieses proximale Applikatorteil dient zur Stoßerzeugung und beim Ausführungsbeispiel als Kollisionspartner für das Projektil. Während eines Stoßes liegt das proximale Applikatorteil an dem distalen Applikatorteil an und vermittelt damit den Stoß weiter. Durch diese Aufteilung kann zum einen die Baugröße des entnehmbaren Applikatorteils begrenzt bleiben. Ferner kann auf der distalen Seite des ersten Applikatorteils ein elastisches Element zum Aufnehmen von Stoßbewegungen vorgesehen sein, insbesondere ein Elastomerschlauchstück oder ein O-Ring, das oder der zudem für die Rückstellung des Applikatorteils in die Ursprungsposition verantwortlich ist. Dieses elastische Element ist dann durch die Aufteilung des Applikators in zumindest zwei Teile beim Ausbau nicht hinderlich.

Gemäß Anspruch 2 ist die bislang beschriebene Kupplung für den Applikator ihrerseits ebenfalls von dem (restlichen) Gerät abnehmbar. Dazu ist sie Teil eines Gehäuseteils, und zwar an einer dem zu behandelnden Körper zugewandten Seite des Gerätes, der dementsprechend vom übrigen Gehäuse abnehmbar ist. Die hierzu vorgesehene lösbare Verbindung zwischen diesem Gehäuseteil und dem übrigen Gehäuse kann bspw. ein Gewinde sein, wie im ersten Ausführungsbeispiel gezeigt. Hierbei sind z. B. Linksgewinde und/oder Gewinde mit besonderer Form, z. B. Rundgewinde oder Trapezgewinde, und/oder mehrgängige Gewinde denkbar. Es kann insbesondere von Vorteil sein, ein sehr spezifisches, gewissermaßen ausgefallenes Gewinde einzusetzen. Grundsätzlich sind nämlich bei vorbekannten einschlägigen Geräten Applikatorkappen vorhanden und über (Rechts-)Gewinde abnehmbar, wobei diese Applikatorkappen einen Applikator umgreifen, an dem Gehäuse halten und durch die Applikatorkappe hindurch nach außen vorstehen lassen. Damit ist ein distales Ende solcher konventioneller Applikatoren auf den zu behandelnden Körper aufsetzbar und kann der Applikator nach Abschrauben der Applikatorkappe entnommen werden. Im Unterschied dazu soll die hier erfindungsgegenständliche Kupplung den Applikator oder ein Applikatorteil ohne Zerlegung des Gehäuses entnehmbar machen, also in einer schnelleren und einfacheren Weise und ohne das Risiko des Eindringens von Schmutz oder des unbeabsichtigten Entfernens weiterer Teile aus dem Gehäuse (etwa O-Ringe, das Projektil etc.).

Die erwähnten ausgefallenen Gewinde haben hierbei den Sinn, die unauthorisierte Verwendung von nicht vom Hersteller für das Gerät vorgesehenen Applikatoren und/oder Applikatorkappen und dadurch entstehende Gefährdungen des Patienten oder Gewährleistungsprobleme zu vermeiden.

Grundsätzlich ist im Fall des Anspruchs 2 vorgesehen, dass neben dem beschriebenen Gehäuseteil mit der Applikatorkupplung darin zu demselben

Gerät auch eine Applikatorkappe (ohne Kupplung) vorliegt. Mit dieser können dann im Austausch gegen das abnehmbare Gehäuseteil z. B. Behandlungen durchgeführt werden, für die Applikatoren günstig sind, die sich schlecht mit der Kupplung montieren lassen. Bspw. zeigt das Ausführungsbeispiel, dass das bereits erwähnte proximale Applikatorteil nach Entfernen des abnehmbaren Gehäuseteils und des distalen Applikatorteils dann nach Aufbringen einer an sich vorbekannten Applikatorkappe auf das Gewinde einsetzbar wäre, nämlich mit seinem distalen Ende, also der flachen Frontfläche. Ansonsten könnten aber natürlich auch andere Applikatoren statt des proximalen Applikatorteils eingesetzt werden und nach Aufschrauben der Applikatorkappe in der an sich bekannten Weise benutzt werden.

Bspw. kann die erfindungsgemäße Kupplung speziell zur Montage von Applikatoren mit besonderer Form des jeweils distalen patientennahen Bereichs eingesetzt werden, die behandlungsabhängig häufig getauscht werden, womit die Kupplung vorteilhaft zur Wirkung kommt. Wenn mit demselben Gerät oder Handstück aber eher "konventionelle" Behandlungen durchgeführt werden sollen, z. B. solche mit Betonung der Druck- bzw. Stoßwelleneinkopplung, möglicherweise geringerem Hub und jedenfalls einfacher mehr oder weniger flacher Frontfläche, so ist dazu die Kupplung vielleicht nicht nötig.

Eine weitere Möglichkeit statt eines Gewindes besteht in einem Bajonettmechanismus zur Verbindung. Dazu kann ein Überlappungsbereich ähnlich wie bei dem Gewinde vorgesehen sein, wobei anstelle des Gewindegangs an einer Seite, z. B. außen, eine bajonetttypische Ausfräsung mit einem axialen Verlauf (parallel zur Stoßrichtung) und einem davon in Umfangsrichtung abknickenden Stück vorgesehen sein kann und auf der anderen Seite, z. B. auf einer inneren Seite und radial nach außen weisend, ein dazu passender Riegel. Dies kann natürlich mehrfach um den Umfang verteilt auftreten. In jedem Fall ist eine Bajonettverbindung eine robuste, sehr schnell zu bedienende und sichere Lösung. Durch einen O-Ring, der beim Anziehen des Bajonetts angedrückt wird, kann eine elastische Vorspannung erzeugt werden, die die Bajonettverbindung zusätzlich sichert. Außerdem kann es eine Verrastung in dem Bajonettmechanismus selbst geben.

Eine weitere Verbindung lässt sich mit einem Spannring lösen, der den Überlappungsbereich umgreift und so angezogen werden kann, dass er sich in seinem Umfang und damit auch radial verkleinert und einen entsprechenden Andruck erzeugt. Zum Spannen des Spannrings ist z. B. ein Exzenterhebel geeignet, den das entsprechende Ausführungsbeispiel zeigt.

Eine weitere Möglichkeit für eine Verbindung verwendet mindestens einen, vorzugsweise zwei Riegelstifte. Diese sind radial, also senkrecht zur Stoßrichtung und von außen nach innen (oder umgekehrt) beweglich und stellen durch Eingriff in ein entsprechendes Gegenstück (eine Ausnehmung) einen Formschluss her. Eine andere vergleichbare Lösung sieht zumindest eine Kugel als Formschlusselement vor, vorzugsweise eine Mehrzahl. Insoweit handelt es sich um eine sehr ähnliche Verbindung wie die eingangs beschriebene Kupplung für den Applikator. Vorzugsweise ist allerdings zur Betätigung eine Betätigungshülse vorgesehen, die nicht in axialer Richtung (Stoßrichtung), sondern in Umfangsrichtung beweglich ist. Sie kann an ihrer Innenseite passende Ausnehmungen für eine Bewegung der Kugel(n) aufweisen und damit ein nach außen Rücken der Kugel(n) und ein Lösen des Formschlusses zu ermöglichen. Auch hierzu wird auf das entsprechende Ausführungsbeispiel verwiesen.

In allen diskutierten Fällen ist vorgesehen, dass Applikatorkappen (ohne Applikatorkupplung) in analoger Weise und im Austausch gegen das abnehmbare Gehäuseteil (mit der Applikatorkupplung) montiert werden können, sodass sich die Flexibilität des Geräts insgesamt erhöht.

Im Folgenden wird die Erfindung anhand eines Ausführungsbeispiels beschrieben, wobei die Offenbarung im Ausführungsbeispiel und auch im vorstehenden Text nicht auf die jeweils dargestellten Merkmalskombinationen beschränkt sein soll, sondern einzelne Merkmale im Rahmen des Hauptanspruchs auch in anderem Zusammenhang oder für sich wichtig sein können. Insbesondere bezieht sich die Offenbarung nicht nur auf das Gerät an sich, sondern auch auf seine Verwendung zur Behandlung, auf seine Verwendung zum Austauschen des Applikators, auf die Verwendung des Applikatorteils zum Ausbauen und Einbauen in das Gerät und auf entsprechende Arbeitsverfahren.
- Figur 1: zeigt einen Längsschnitt durch ein vorderes Gehäuseteil eines erfindungsgemäßen Geräts mit einer Applikatorkupplung;
- Figur 2: zeigt eine perspektivische Darstellung eines zweiten Ausführungsbeispiels, das von dem ersten hinsichtlich der mechanischen Verbindung zwischen dem die Schnellkupplung enthaltenden Gehäuseteil und dem übrigen Gehäuse abweicht, nämlich mit einer Bajonettverbindung;
- Figur 3: zeigt ebenfalls perspektivisch und im Schnitt ein drittes Ausführungsbeispiel mit einer Exzenter-Spannringverbindung;
- Figur 4: zeigt ein viertes Ausführungsbeispiel mit einer Riegelstiftverbindung, ebenfalls perspektivisch und im Schnitt;
- Figur 5: zeigt als letztes Ausführungsbeispiel eine Formschlussverbindung mit Kugeln perspektivisch und im Schnitt.

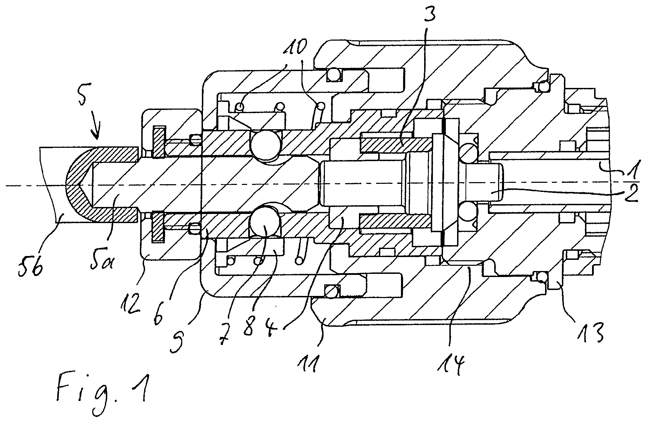

Der dem ersten Ausführungsbeispiel zugrunde liegende Typ eines pneumatisch betriebenen Druckwellengeräts ist hinlänglich bekannt, wozu auf den eingangs zitierten Stand der Technik verwiesen werden kann. Er weist eine Basisstation mit einer Pneumatikversorgung, eine davon ausgehende Versorgungsleitung und am Ende der Versorgungsleitung ein mobiles und mit der Hand handhabbares Handstück auf, das hier als das Gerät bezeichnet wird. Die Pneumatikversorgung ist über die Leitung und ein entsprechendes Schaltventil an ein Beschleunigungsrohr angeschlossen, dessen in der Darstellung der Figur linkes Ende in der Figur 1 eingezeichnet und mit 1 beziffert ist. Der Pneumatikanschluss findet sich am nicht dargestellten rechten Ende desselben Rohres 1 und in dem Rohr 1 ist ein nicht dargestelltes und den Querschnitt des Rohres ausfüllendes Projektil bewegbar, das am Ende seiner Bewegungsstrecke auf die rechte Stirnfläche eines proximalen Applikatorteils 2 aufprallen kann.

Dieses proximale Applikatorteil 2 hat eine im Vergleich zum Stand der Technik weitgehend konventionelle Form und stützt sich mit einem radial vergrößerten Flansch und dessen distaler Schulterseite an einem Elastomerschlauchstück 3 ab, das bei den Kollisionen verformbar ist und eine Bewegung des proximalen Applikatorteils 2 im Bereich über einem Millimeter zulassen kann. Es ist ferner für die Rückstellung verantwortlich und entkoppelt die Schläge vom Gerätegehäuse. An der distalen Seite läuft dieses proximale Applikatorteil 2 in einem zylindrischen Stift aus, der eine Führungsmanschette 4 durchsetzt und zur distalen Seite etwas darüber hinausragt. Bei konventionellen Geräten wäre die distale Stirnfläche dieses Stifts die Kontaktfläche des Applikators zum Patienten und die Führungsmanschette 4 in einer (oder als) abschraubbaren Kappe realisiert. Man müsste also die Kappe abnehmen und damit eine Teilzerlegung vornehmen, bevor man den Applikator (in diesem Fall das proximale Applikatorteil 2) entnehmen kann.

Im vorliegenden Fall ist eine äußere Betätigungshülse 11 aufgeschraubt auf ein weiter innen und "proximaler" liegendes Flanschelement 13, wobei das Gewinde mit 14 beziffert ist. Man erkennt ferner etwas proximal von dem Gewinde 14 eine O-Ringabdichtung. In dieser Form lässt sich mit der Betätigungshülse 11 das gesamte vordere Gehäuseteil einschließlich der Kupplung abnehmen und wird das proximale Applikatorteil 2 zugänglich. Hierauf wird weiter unten noch näher eingegangen.

Figur 1 zeigt ferner, dass sich die linke Stirnfläche des proximalen Applikatorteils 2 in Kontakt mit der rechten Stirnfläche eines distalen Applikatorteils 5 befindet. Dieses distale Applikatorteil 5 ist seinerseits zweiteilig und besteht hier aus einem ersten Teil 5a und einem zweiten Teil 5b. Figur 1 zeigt das Teil 5b nicht mehr vollständig, weil es auf die für die Behandlung ausschlaggebende Form dieses Teils 5b im vorliegenden Zusammenhang nicht ankommt. Beide Teile 5a und 5b können fest verbunden sein, bspw. durch eine Schraubverbindung, Schweißverbindung oder Pressverbindung. Die Aufteilung in zwei Teile hat hier produktionstechnische Gründe, ein einstückiges distales Applikatorteil 5 wäre aber genauso gut möglich.

Jedenfalls ist das distale Applikatorteil 5, konkret sein erstes Teil 5a, in einem Aufnahmestück 6 geführt, und zwar formschlüssig, z. B hexagonal, und damit gegenüber diesem Aufnahmestück 6 gegen Verdrehung gesichert. Das Aufnahmestück 6 seinerseits muss entsprechend fest verdrehgesichert im übrigen Gehäuse gehalten sein, wozu entsprechende in Figur 1 nach rechts weisende Stirnflächen oder nach außen weisende Mantelflächen in reibungssteigernder Form beschichtet oder oberflächenstrukturiert sein können. Um gleichzeitig ein Fressen zwischen dem distalen Applikatorteil 5 und dem Aufnahmestück 6 zu verhindern, können entsprechende Materialpaarungen verwendet werden, z. B. applikatorseitig rostfreier Edelstahl und aufnahmeseitig Bronze, hochfester Kunststoff (z. B. PEEK) oder Keramik.

Figur 1 zeigt ferner, dass in dem Aufnahmestück 6 (in Figur 1 oben und unten) Öffnungen für Formschlusskugeln 7 vorgesehen sind, die nach innen in entsprechende außenseitige Nuten an dem ersten 5a der beiden distalen Applikatorteile eingreifen. Diese Nuten sind in Figur 1 nach rechts länger als nötig, so dass sich der gesamte Applikator 5 und 6 bei einem Stoß nach links bewegen kann. Andererseits verhindert der Formschluss zwischen den Kugeln 7 und den dazugehörenden Nuten ein Herausfallen oder -schießen des distalen Applikatorteils aus dem Gehäuse. Das distale Applikatorteil kann vielmehr z. B. einfach durch Aufdrücken auf dem Patienten nach einem Stoß wieder in seine gezeichnete Ursprungslage zurückgeschoben werden.

Radial außerhalb der Kugeln 7 erkennt man eine Klemmhülse 8, die ersichtlich an ihrem linken Ende eine innere Schrägfläche aufweist. Wenn diese Klemmhülse 8 aus der gezeichneten Position heraus nach rechts verschoben wird, können die Kugeln 7 radial nach außen weichen und kann das distale Applikatorteil 5 insgesamt einfach nach links aus dem Gehäuse herausgezogen werden. Für dieses Verschieben der Klemmhülse 8 sorgt ein händisches (nach rechts) Hineindrücken eines von außen zugänglichen Gehäuseteils 9, wobei eine Schraubenfeder 10 dagegendrückt bzw. für die Rückführbewegung sorgt und dieses verschiebbare Gehäuseteil 9 in der Betätigungshülse 11 des Gehäuses verschiebbar und durch einen O-Ring gehemmt geführt ist. Diese Betätigungshülse 11 umgreift und hält auch das bereits erwähnte Aufnahmestück 6.

Die Kugeln 7 sind übrigens in radialer Richtung nicht kraftbeaufschlagt an sich, sondern werden verdrängt, wenn der Benutzer das distale Applikatorteil 5 herauszieht. Da es sich nur um zwei (oder in anderen Fällen um z. B. drei, vier oder sechs) Kugeln handelt, für die jeweils eigene Nuten vorgesehen sind, tragen sie auch zur Verdrehsicherung bei; wesentlich für die Verdrehsicherung ist aber die beschriebene Mehrkant-Formschluss-Ausführung der Teile 5a außen und 6 innen.

In Figur 1 links von dem verschiebbaren und zur Betätigung der Kupplung dienenden Gehäuseteil 9 findet sich noch eine Sicherungsmutter 12, die auf dem Aufnahmeteil 6 aufgeschraubt und gegenüber diesem über einen O-Ring und gegenüber dem Teil 5a über einen Elastomerflachring abgedichtet ist. Dabei ist die Anlage zwischen dem Elastomerflachring (an dessen Innenrand) und dem hier hexagonalen ersten Teil 5a des distalen Applikatorteils gleitfähig.

Insgesamt erkennt man in Figur 1, dass ein Projektilstoß den gesamten Applikator nach links versetzt und dabei eine entsprechende Stoßbewegung des nicht gezeichneten distalsten Endes des Applikators (ganz links) gegen den Patientenkörper zur Folge hat, wobei die Einkopplung einer eigentlichen Druckwelle parallel auftreten wird, aber in vielen Fällen gar nicht wesentlich ist. Die Druckwelle wird dabei übrigens in dem proximalen Applikatorteil 2 erzeugt und über dieses und die beiden weiteren Applikatorteile 5a und 5b in den Körper übertragen. Nach dem Stoß kehrt das proximale Applikatorteil 2 infolge der Kraftbeaufschlagung durch das Elastomerschlauchstück 3 selbsttätig nach rechts zurück; für das distale Applikatorteil 5a und 5b folgt dies aus der Druckauflage auf den Patientenkörper.

Wenn das distale Applikatorteil 5 gewechselt werden soll, schiebt der Benutzer das verschiebbare Gehäuseteil 9 nach rechts und damit die Klemmhülse 4 ebenfalls, sodass die Kugeln 7 radial nach außen gelangen können. Dabei sind übrigens die Öffnungen in dem Aufnahmestück 6 für die Kugeln 7 radial innen zu eng, sodass die Kugeln nicht herausfallen können. Beim Wiedereinsetzen eines anderen entsprechenden distalen Applikatorteils muss wieder das Gehäuseteil 9 nach rechts geschoben werden, um die Kugeln nach außen verdrängen zu können. Wenn das distale Applikatorteil 5 vollständig eingesetzt ist, kann man das Gehäuseteil 9 loslassen und verriegelt die Kupplung.

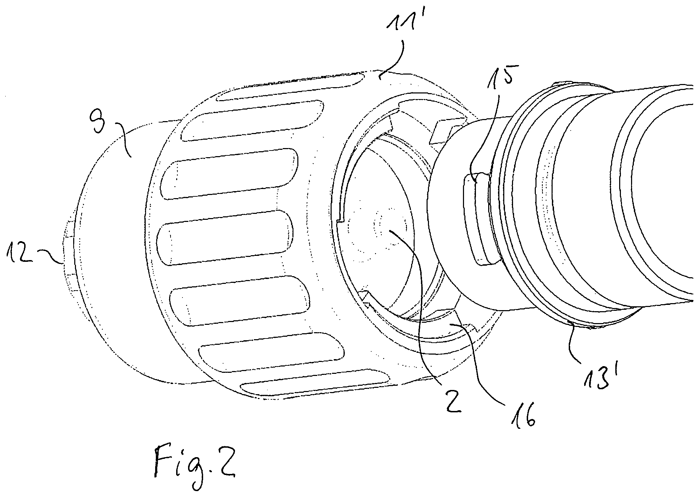

Figur 2 zeigt in perspektivischer Ansicht ein zweites Ausführungsbeispiel, hier ohne distales Applikatorteil 5. Dabei ist die beim ersten Ausführungsbeispiel in Figur 1 durch das Gewinde 14, das bspw. ein Linksgewinde oder in anderer Weise ausgefallenes Gewinde sein kann, realisierte Verbindung durch einen Bajonettmechanismus gebildet. Im Übrigen entsprechen sich die Ausführungsbeispiele, wobei zur Verdeutlichung einige Bezugszeichen aus Figur 1 eingetragen sind. Die entsprechenden Elemente werden aber nicht erneut erläutert.

Man erkennt an einer Außenfläche eines gegenüber Figur 1 etwas modifizierten Flanschteils 13' einen von drei über den Umfang verteilten Bajonettriegeln 15 und an einer Innenfläche einer ebenfalls gegenüber Figur 1 modifizierten Betätigungshülse 11' dementsprechende Riegelelemente 16, die zwischen sich für die Bajonettriegel 15 passende Ausnehmungen freilassen und auf der distalen Seite Platz zum Verdrehen des in Figur 2 getrennt gezeigten abnehmbaren vorderen Gehäuseteils (9, 11', 12, ...) gegenüber dem übrigen Gehäuse (13', ...) lassen. Dabei können natürlich Anschläge hinter den Riegelelementen 16 gebildet sein, an die die Bajonettriegel 15 bei diesem Verdrehen anstoßen und es kann ferner eine Verrastung vorgesehen sein. Schließlich ist in ähnlicher Weise wie in Figur 1 auf der distalen Seite des radial nach außen vorstehenden Vorsprungs (dort wo das Bezugszeichen 13' angezeichnet ist) ein umlaufender O-Ring angebracht, gegen den der Bajonettmechanismus eine elastische Andruckkraft erzeugt. Man erkennt in Figur 2, dass nach Abnehmen des distalen Gehäuseteils insbesondere das proximale Applikatorteil 2 zugänglich ist und ausgebaut werden kann. Ferner kann eine ohne Schnellkupplung ausgestattete (nicht gezeichnete) Applikatorkappe mit im Wesentlichen der äußeren Gestalt der Betätigungshülse 11', aber angepasst an die Aufnahme vorbekannter Applikatoren (analog 2) angebracht werden.

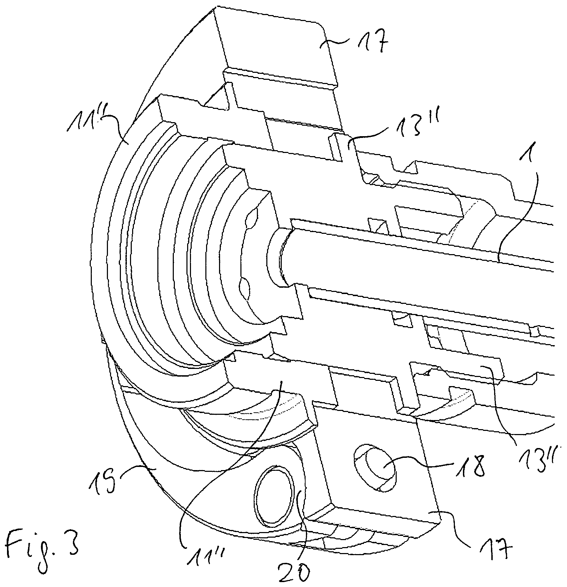

Figur 3 zeigt ein weiteres Ausführungsbeispiel mit einem Spannringmechanismus anstelle des Bajonettmechanismus aus Figur 2. In der Figur ist das "übrige Gehäuseteil" zusammen mit dem Spannring 17 und einem proximalen Teil 11" des abnehmbaren vorderen Gehäuseteils dargestellt. Auch hier erleichtern die Bezugszeichen einen Vergleich mit Figur 1. Das Flanschteil 13 aus Figur 1 ist nun geringfügig modifiziert, trägt nämlich kein Außengewinde 14, und ist damit als 13" beziffert. Auf einer zylindrischen Außenfläche sitzt eine zylindrische Innenfläche des (hier für das abnehmbare vordere Gehäuseteil stehenden) Hülsenteils 11" und ist durch den Spannring 17 darauf gepresst. Der Spannring 17 hat im Wesentlichen die Gestalt einer Schelle und ist (vollständig gedacht) links hinten, oben und (nicht gezeigt) rechts vorn geschlossen, also nur unten in der bezeichneten Form geteilt. Dabei durchsetzt ein Zapfen 18 die Teilungsstelle und ist fest mit dem nicht gezeigten gegenüberliegenden Spannringteil verbunden. Dieser Zapfen 18 kann mit dem gezeichneten Exzenterhebel 19 angezogen werden, indem dieser Exzenterhebel 19 aus einer nicht gezeigten nach unten weisenden Stellung in die gezeigte angelegte Stellung überführt wird. Dabei presst sich ein Exzenterabschnitt 20 des Exzenterhebels 19 gegen eine entsprechende Anlagefläche.

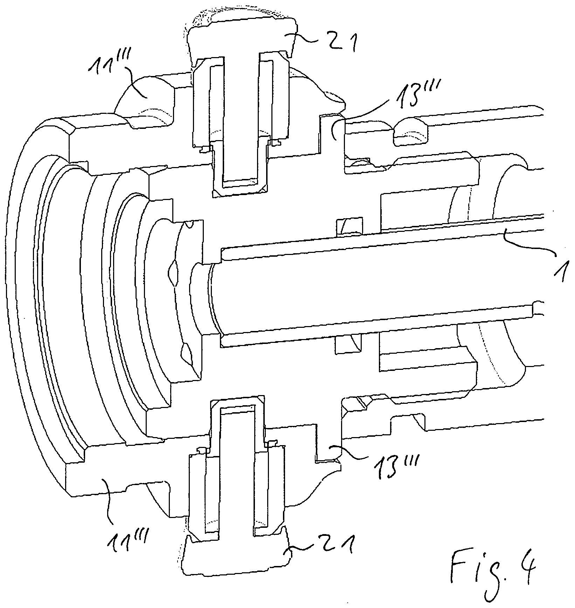

Das nächste Ausführungsbeispiel in Figur 4 zeigt einen Riegelstiftmechanismus mit Riegelstiften 21 in einem Hülsenteil 11"'. Dieses Hülsenteil 11"' sitzt erneut mit einer im Wesentlichen zylindrischen Innenfläche auf einer im Wesentlichen zylindrischen Außenfläche eines modifizierten Flanschteils 13"' auf, wobei die Situation deutliche Ähnlichkeiten zum vorherigen Ausführungsbeispiel hat. Die zylindrische Außenfläche des Flanschteils 13"' weist entsprechende Sacklöcher für das Eindringen der nach innen weisenden Vorderteile der Riegel 21 auf. Die Riegel 21 sind jeweils durch nicht gezeichnete Schraubenfedern um den Schaft und zwischen dem Betätigungskopf und den nach innen weisenden Teilen federbeaufschlagt, rutschen also selbsttätig in die erwähnten Sacklöcher hinein und müssen händisch daraus zurückgezogen werden, um das vordere Gehäuseteil (11"' ...) von dem restlichen Gehäuse abzuziehen. Im vorliegenden Fall sind zwei solche Riegelstifte 21 vorgesehen.

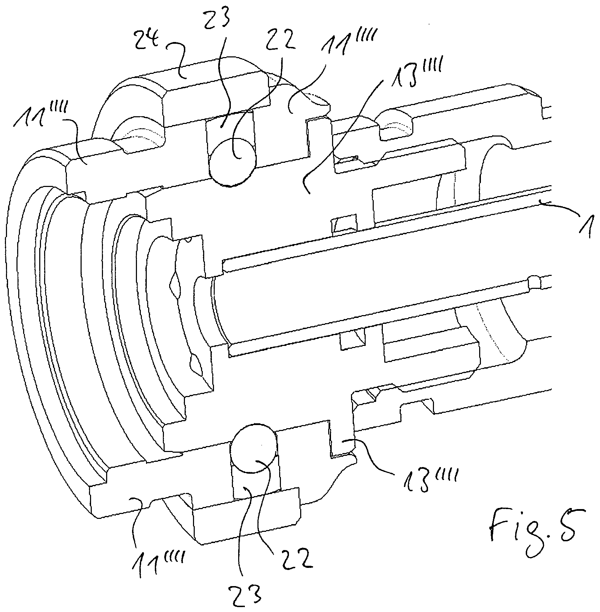

Das letzte Ausführungsbeispiel zeigt Figur 5. Es kann weitgehend auf die vorherigen Erläuterungen zu Figur 4 verwiesen werden. Anstelle der Riegelstifte 21 sind hier Kugeln 22 als Formschlusselement vorgesehen und in entsprechende sphärische Ausnehmungen an der zylindrischen Außenseite eines Flanschteils 13"" hineingedrückt, das im Übrigen dem bereits erläuterten Flanschteil 13"' entspricht. Die Kugeln sind gehalten in zylindrischen radialen Bohrungen in einem Hülsenteil 11"". Radial außerhalb der Kugeln 22 findet sich jeweils ein Zwischenelement 23 mit an die Kugel angepasster Innenfläche, das von einem äußeren Betätigungshülsenteil 24 daran gehindert wird, weiter nach außen zu gelangen. Diese Betätigungshülse 24 kann gegenüber dem Hülsenteil 11"" in Umfangsrichtung verdreht werden, was nicht gezeichnete Ausnehmungen an seiner Innenseite zur Überdeckung mit den Zwischenelementen 23 bringt und damit ein radiales nach außen Gleiten dieser Zwischenelemente 23 und infolgedessen der Kugeln 22 ermöglicht. Eine Führung der Betätigungshülse 24 hinsichtlich der Drehbewegung ist nicht gezeichnet, aber vorzusehen. Sie kann ferner so federbeaufschlagt werden, dass sie von selbst in die Verriegelungsposition gelangt und aus dieser gegen die Federkraft verdreht werden muss.

## Patentansprüche

1. Gerät zur Behandlung des menschlichen oder tierischen Körpers mit
- einem Applikator (2, 5) zum Auflegen auf den Körper von außen,
- einem Gehäuse (9, 11-11"", 12), in dem der Applikator (2, 5) gehalten ist, und
- einem Mechanismus (1) zum Erzeugen von Stößen des Applikators (2, 5) relativ zu dem Gehäuse (9, 11-11"", 12) in einer Stoßrichtung, so dass die Stöße bei dem Auflegen in den Körper eingekoppelt werden können,
wobei, dass der Applikator (2, 5) in dem Gehäuse (9, 11-11"", 12) in einer Kupplung (7, 8, 9, 10) gehalten ist
und zumindest ein distales Applikatorteil (5) nach Lösen der Kupplung (7, 8, 9, 10) aus der Kupplung (7, 8, 9, 10) und dem Gehäuse (9, 11-11ʺʺ, 12) nach vorn herausziehbar ist, ohne dazu ein weiteres Gehäuseteil abzubauen, und
wobei die Kupplung (7, 8, 9, 19) lösbar ist durch Verschieben einer Klemmhülse (8), die ausgelegt ist zum Halten des Applikators (2, 5) durch Beaufschlagen von zumindest einem Formschlusselement (7) nach innen auf den Applikator (2, 5) zu in einer zu der Stoßrichtung senkrechten Richtung und zum Freigeben durch Zulassen einer Rückbewegung des Formschlusselements (7),
**dadurch gekennzeichnet, dass** das zumindest eine Formschlusselement (7) in eine applikatorseitige Nut eingreift, die dem Formschlusselement (7) Raum für eine Stoßbewegung des Applikators (2, 5) nach vorn lässt.

2. Gerät zur Behandlung des menschlichen oder tierischen Körpers mit
- einem Applikator (2, 5) zum Auflegen auf den Körper von außen,
- einem Gehäuse (9, 11-11"", 12), in dem der Applikator (2, 5) gehalten ist, und
- einem Mechanismus (1) zum Erzeugen von Stößen des Applikators (2, 5) relativ zu dem Gehäuse (9, 11-11"", 12) in einer Stoßrichtung, so dass die Stöße bei dem Auflegen in den Körper eingekoppelt werden können,
wobei der Applikator (2, 5) in dem Gehäuse (9, 11-11"", 12) in einer Kupplung (7, 8, 9, 10) gehalten ist
und zumindest ein distales Applikatorteil (5) nach Lösen der Kupplung (7, 8, 9, 10) aus der Kupplung (7, 8, 9, 10) und dem Gehäuse (9, 11-11"", 12) nach vorn herausziehbar ist, ohne dazu ein weiteres Gehäuseteil abzubauen, und
der Applikator (2, 5) zumindest zweiteilig ist, wobei ein distales Applikatorteil (5) nach Lösen der Kupplung (7, 8, 9, 10) herausziehbar ist und ein proximales Applikatorteil (2) in dem Gehäuse verbleibt und wobei das proximale Applikatorteil (2) den Stoß auf das distale Applikatorteil (5) überträgt,
**dadurch gekennzeichnet, dass** die Kupplung (7, 8, 9, 10) in einem dem zu behandelnden Körper zugewandten Teil (7-10, 11-11"", 12) des Gehäuses vorgesehen ist, welches Gehäuseteil (7-10, 11-11"", 12) durch Lösen einer mechanischen Verbindung (15-24) zusammen mit der Kupplung (7, 8, 9, 10) von dem übrigen Gehäuse (13-13ʺʺ) abgenommen werden kann,
und **gekennzeichnet durch** eine zusätzliche Applikatorkappe als Gehäuseteil, die statt des die Kupplung (7, 8, 9, 10) aufweisenden Gehäuseteils (7-10, 11-11ʺʺ, 12) über eine der mechanischen Verbindung (15-24) entsprechende mechanische Verbindung an dem übrigen Gehäuse (13-13ʺʺ) befestigt werden kann und dazu ausgelegt ist, in diesem befestigten Zustand den proximalen Applikatorteil (2) oder einen an dessen Stelle eingesetzten Applikator in der Stoßrichtung durch die Applikatorkappe hindurch nach außen vorstehen und zur Anlage auf dem zu behandelnden Körper kommen zu lassen.

3. Gerät nach Anspruch 1 oder 2, bei dem der Mechanismus (1) zum Erzeugen der Stöße ein Projektil und eine Einrichtung (1) zum Beschleunigen des Projektils in solcher Weise aufweist, dass das Projektil auf den Applikator (2, 5) schlägt und dadurch den Stoß erzeugt, vorzugsweise eine pneumatische Einrichtung zur Beschleunigung des Projektils.

4. Gerät nach einem der vorstehenden Ansprüche, bei dem der Applikator (2, 5) in dem Gerät durch einen Formschluss verdrehsicher gehalten ist, und zwar bezüglich Rotationen um eine zur Stoßrichtung parallele Drehachse, wobei vorzugsweise applikatorseitig an dem Formschluss ein Mehrkantstift (5a) teilnimmt, der sich in Stoßrichtung erstreckt, und auf der anderen Seite eine dazu passende Aufnahme (6).

5. Gerät nach Anspruch 2, optional in Verbindung mit Anspruch 3 oder 4, bei dem die Kupplung (7, 8, 9, 19) lösbar ist durch Verschieben einer Klemmhülse (8), die ausgelegt ist zum Halten des Applikators (2, 5) durch Beaufschlagen von zumindest einem Formschlusselement (7) nach innen auf den Applikator (2, 5) zu in einer zu der Stoßrichtung senkrechten Richtung und zum Freigeben durch Zulassen einer Rückbewegung des Formschlusselements (7).

6. Gerät **nach Anspruch 5,** bei dem die Kupplung (7, 8, 9, 10) zum Lösen betätigt werden kann durch Verschieben eines Gehäuseteils (9) des Geräts gegen eine Federkraft, und zwar vorzugsweise entgegen der Stoßrichtung, und das Gehäuseteil (9) die Klemmhülse (8) verschiebt oder aufweist und die Klemmhülse (8) vorzugsweise Schrägflächen zum Beaufschlagen des Formschlusselements aufweist, und wobei als Formschlusselement (7) vorzugsweise mindestens zwei Kugeln vorgesehen sind.

7. Gerät nach Anspruch 2 und 6, optional auch in Verbindung mit Anspruch 3, 4 oder 5, bei dem das zumindest eine Formschlusselement (7) in eine applikatorseitige Nut eingreift, die dem Formschlusselement (7) Raum für eine Stoßbewegung des Applikators (2, 5) nach vorn lässt.

8. Gerät nach Anspruch 1, optional mit einem der Ansprüche 3 bis 7, bei dem der Applikator (2, 5) zumindest zweiteilig ist, wobei ein distales Applikatorteil (5) nach Lösen der Kupplung (7, 8, 9, 10) herausziehbar ist und ein proximales Applikatorteil (2) in dem Gehäuse verbleibt und wobei das proximale Applikatorteil (2) den Stoß auf das distale Applikatorteil (5) überträgt, wobei vorzugsweise das proximale Applikatorteil (2) vor der Erzeugung eines Stoßes an einem elastischen Element (3) zwischen diesem proximalen Applikatorteil (2) und einem anderen Geräteteil (4) anliegt, welches eine Stoßbewegung des proximalen Applikatorteils (2) begrenzt und dieses in die Ausgangslage zurückführt.

9. Gerät nach einem der vorstehenden Ansprüche, bei dem die Kupplung (7, 8, 9, 10) in einem dem zu behandelnden Körper zugewandten Teil (7-10, 11', 12) des Gehäuses vorgesehen ist, welches Gehäuseteil (7-10, 11', 12) durch Lösen einer Bajonettverbindung (15, 16) zusammen mit der Kupplung (7, 8, 9, 10) von dem übrigen Gehäuse (13') abgenommen werden kann.

10. Gerät nach einem der Ansprüche 1 bis 8, bei dem die Kupplung (7, 8, 9, 10) in einem dem zu behandelnden Körper zugewandten Teil (7-10, 11", 12) des Gehäuses (9, 11, 12) vorgesehen ist, welches Gehäuseteil (7-10, 11", 12) durch Lösen einer Spannringverbindung (17-20) zusammen mit der Kupplung (7, 8, 9, 10) von dem übrigen Gehäuse (13") abgenommen werden kann, wobei die Spannringverbindung (17-20) einen Spannring (17-20) aufweist, der einen Überlappungsbereich zwischen dem Gehäuseteil (7-10, 11", 12) und dem übrigen Gehäuse (13") umgreift.

11. Gerät nach Anspruch 10, bei dem der Spannring (17-20) über einen Exzenterhebel (19) spannbar ist.

12. Gerät nach einem der Ansprüche 1 bis 8, bei dem die Kupplung (7, 8, 9, 10) in einem dem zu behandelnden Körper zugewandten Teil (7-10, 11‴, 12) des Gehäuses vorgesehen ist, welches Gehäuseteil (7-10, 11‴, 12) durch Lösen einer Riegelstiftverbindung (21) zusammen mit der Kupplung (7, 8, 9, 10) von dem übrigen Gehäuse (13‴) abgenommen werden kann, wobei die Riegelstiftverbindung (21) zumindest einen, vorzugsweise zwei Riegelstifte (21) aufweist, die senkrecht zu Stoßrichtung beweglich und dazu ausgelegt sind, in einem Überlappungsbereich zwischen dem Gehäuseteil (7-10, 11‴, 12) und dem übrigen Gehäuse (13‴) einen Verriegelungsformschluss herzustellen.

13. Gerät nach einem der Ansprüche 1 bis 8, bei dem die Kupplung (7, 8, 9, 10) in einem dem zu behandelnden Körper zugewandten Teil (7-10, 11"", 12) des Gehäuses vorgesehen ist, welches Gehäuseteil (7-10, 11"", 12) durch Lösen einer Formschlussverbindung (22-24) zusammen mit der Kupplung (7, 8, 9, 10) von dem übrigen Gehäuse (13"") abgenommen werden kann, wobei die Formschlussverbindung (22-24) zumindest eine Kugel (22) als Formschlusselement aufweist, die durch eine Bewegung einer Betätigungshülse (24) in einem Überlappungsbereich des Gehäuseteils (7-10, 11"", 12) und des übrigen Gehäuses (13ʺʺ) senkrecht zur Stoßrichtung bewegt werden kann und damit einen durch Eingriff der Kugel (22) in eine passende Ausnehmung entstehenden Formschluss herstellt und aufhebt.

14. Gerät nach Anspruch 13, bei dem die Betätigungshülse (24) in einer Umfangsrichtung um die Stoßrichtung herum beweglich ist und an einer Innenseite eine Ausnehmung für eine Bewegung der zumindest einen Kugel (22) aufweist.

15. Gerät nach Anspruch 1 und 8, bei dem die Kupplung (7, 8, 9, 10) in einem dem zu behandelnden Körper zugewandten Teil (7-10, 11-11"", 12) des Gehäuses vorgesehen ist, welches Gehäuseteil (7-10, 11-11"", 12) durch Lösen einer mechanischen Verbindung (15-24) zusammen mit der Kupplung (7, 8, 9, 10) von dem übrigen Gehäuse (13-13"") abgenommen werden kann, wobei die mechanische Verbindung (15-24) vorzugsweise einem der Ansprüche 10 bis 14 entspricht
und mit einer zusätzlichen Applikatorkappe als Gehäuseteil, die statt des die Kupplung (7, 8, 9, 10) aufweisenden Gehäuseteils (7-10, 11-11"", 12) über eine der mechanischen Verbindung (15-24) entsprechende mechanische Verbindung an dem übrigen Gehäuse (13-13ʺʺ) befestigt werden kann und dazu ausgelegt ist, in diesem befestigten Zustand den proximalen Applikatorteil (2) oder einen an dessen Stelle eingesetzten Applikator in der Stoßrichtung durch die Applikatorkappe hindurch nach außen vorstehen und zur Anlage auf dem zu behandelnden Körper kommen zu lassen.

## Claims

1. An apparatus for treating a human or animal body, having
- an applicator (2, 5) for being placed on said body from the outside,
- a housing (9, 11-11"", 12) in which said applicator (2, 5) is held, and
- an apparatus (1) for generating strokes of said applicator (2, 5) with respect to said housing (9, 11-11"", 12) in a stroke direction so that said strokes can be coupled into said body when said applicator is placed on said body,
wherein said applicator (2, 5) is held in said housing (9, 11-11"", 12) in a coupling (7, 8, 9, 10)
and at least a distal applicator part (5) can be pulled out of said coupling (7, 8, 9, 10) and said housing (9, 11-11"", 12) to the front after releasing said coupling (7, 8, 9, 10), that is without a further housing part has to be removed, and
wherein said coupling (7, 8, 9, 10) can be released by displacing a split ring (8) adapted for holding said applicator (2, 5) by acting on at least one form closure element (7) inwards towards said applicator (2, 5) in a direction perpendicular to said stroke direction and for releasing by allowing a backward motion of said form closure element (7),
**characterized in that** said at least one form closure element (7) engages in a groove on the applicator side, said groove providing space to said form closure element (7) for a stroke movement of said applicator (2, 5) towards the front.

2. An apparatus for treating a human or animal body, having
- an applicator (2, 5) for being placed on said body from the outside,
- a housing (9, 11-11"", 12) in which said applicator (2, 5) is held, and
- an apparatus (1) for generating strokes of said applicator (2, 5) with respect to said housing (9, 11-11"", 12) in a stroke direction so that said strokes can be coupled into said body when said applicator is placed on said body,
wherein said applicator (2, 5) is held in said housing (9, 11-11"", 12) in a coupling (7, 8, 9, 10)
and at least a distal applicator part (5) can be pulled out of said coupling (7, 8, 9, 10) and said housing (9, 11-11 "", 12) to the front after releasing said coupling (7, 8, 9, 10), that is without a further housing part has to be removed, and
said applicator (2, 5) is at least two-part, wherein a distal applicator part (5) can be pulled out after releasing said coupling (7, 8, 9, 10) and a proximal applicator part (2) remains in said housing, said proximal applicator part (2) transmitting said stroke onto said distal applicator part (5),
**characterized in that** said coupling (7, 8, 9, 10) is provided in a housing part (7-10, 11-11"", 12) of said housing, which faces said body to be treated, wherein said housing part (7-10, 11-11 "", 12) can be removed together with said coupling (7, 8, 9, 10) from the remaining housing (13-13"") by releasing a mechanical connection (15-24),
and **characterized by** an additional applicator cap as a housing part, which can be mounted at said remaining housing (13-13"") instead of said housing part (7-10, 11-11"", 12) comprising said coupling (7, 8, 9, 10) by a mechanical connection corresponding to said mechanical connection (15-24), said applicator cap being adapted for, in the state of being mounted, letting the proximal applicator part (2) or an applicator inserted instead of the latter protrude through said applicator cap in said stroke direction outwards for contacting said body to be treated.

3. The apparatus according to claim 1 or 2, wherein said apparatus (1) for generating said strokes comprises a projectile and a device (1) for accelerating said projectile in such a way that said projectile hits said applicator (2, 5) and generates said stroke, thus, preferably a pneumatic device for accelerating said projectile.

4. The apparatus according to one of the preceding claims, wherein said applicator (2, 5) is held in said apparatus in a manner prevented from rotating by a form closure, namely with respect to rotations around a rotational axis parallel to said stroke direction, wherein a polygonal stud (5a) extending in said stroke direction participates in said form closure preferably on the applicator side, a receiver (6) fitting thereto participating on the other side.

5. The apparatus according to claim 2, optionally in combination with claim 3 or 4, wherein said coupling (7, 8, 9, 10) can be released by displacing a split ring (8) adapted for holding said applicator (2, 5) by acting on at least one form closure element (7) inwards towards said applicator (2, 5) in a direction perpendicular to said stroke direction and for releasing by allowing a backward motion of said form closure element (7).

6. The apparatus according to claim 5, wherein said coupling (7, 8, 9, 10) can be operated for said releasing by displacing a housing part (9) of said apparatus against a spring force, namely preferably against said stroke direction, wherein said housing part (9) displaces or comprises said split ring (8), said split ring (8) preferably comprising oblique faces for said acting on said form closure elements, wherein preferably at least two spheres are provided as said form closure element (7).

7. The apparatus according to claim 2 and 6, optionally also in combination with claim 3, 4 or 5, wherein said at least one form closure element (7) engages in a groove on the applicator side, said groove providing space to said form closure element (7) for a stroke movement of said applicator (2, 5) towards the front.

8. The apparatus according to claim 1, optionally also in combination with one of claims 3 to 7, wherein said applicator (2, 5) is at least two-part, wherein a distal applicator part (5) can be pulled out after releasing said coupling (7, 8, 9, 10) and a proximal applicator part (2) remains in said housing, said proximal applicator part (2) transmitting said stroke onto said distal applicator part (5), wherein preferably said proximal applicator part (2) contacts an elastic element (3) between said proximal applicator part (2) and another apparatus part (4) prior to said generation of said stroke, which defines a stroke movement of said proximal applicator part (2) and guides the latter back into its initial position.

9. The apparatus according to one of the preceding claims, wherein said coupling (7, 8, 9, 10) is provided in a housing part (7-10, 11', 12) of said housing, which faces said body to be treated, said housing part (7-10, 11', 12) being removable together with said coupling (7, 8, 9, 10) from the remaining housing (13') by releasing a bayonet connection (15, 16).

10. The apparatus according to one of claims 1 to 8, wherein said coupling (7, 8, 9, 10) is provided in a housing part (7-10, 11", 12) of said housing (9, 11, 12), which faces said body to be treated, said housing part (7-10, 11", 12) being removable together with said coupling (7, 8, 9, 10) from the remaining housing (13") by releasing a locking ring connection (17-20), wherein said locking ring connection (17-20) comprises a locking ring (17-20) which encloses an overlap region between said housing part (7-10, 11", 12) and said remaining housing (13").

11. The apparatus according to claim 10, wherein said locking ring (17-20) can be locked by an eccentric lever (19).

12. The apparatus according to one of claims 1-8, wherein said coupling (7, 8, 9, 10) is provided in a housing part (7-10, 11‴, 12) of said housing, which faces said body to be treated, wherein said housing part (7-10, 11‴, 12) can be removed together with said coupling (7, 8, 9, 10) from the remaining housing (13‴) by releasing a locking pin connection (21), wherein said locking pin connection (21) comprises at least one, preferably two locking pins which can be moved perpendicularly to said stroke direction and are adapted for forming a locking form closure in an overlap region between said housing part (7-10, 11‴, 12) and said remaining housing (13‴).

13. The apparatus according to one of claims 1-8, wherein said coupling (7, 8, 9, 10) is provided in a housing part (7-10, 11"", 12) of said housing, which faces said body to be treated, wherein said housing part (7-10, 11"", 12) can be removed together with said coupling (7, 8, 9, 10) from the remaining housing (13"") by releasing a form closure connection (22-24), wherein said form closure connection (22-24) comprises at least one sphere (22) as a form closure element, which can be moved perpendicularly to said stroke direction by a movement of an actuation sleeve (24) in an overlap region of said housing part (7-10, 11"", 12) and said remaining housing (13""), whereby a form closure formed by said sphere (22) engaging in a corresponding recess is formed and released.

14. The apparatus according to claim 13, wherein said actuation sleeve (24) can be moved in a circumferential direction around said stroke direction and comprises a recess on an inside for moving said at least one sphere (22).

15. The apparatus according to claim 1 and 8, wherein said coupling (7, 8, 9, 10) is provided in a housing part (7-10, 11-11"", 12) of said housing, which faces said body to be treated, wherein said housing part (7-10, 11-11 "", 12) can be removed together with said coupling (7, 8, 9, 10) from the remaining housing (13-13"") by releasing a mechanical connection (15-24), said mechanical connection (15-24) corresponding preferably to one of claims 10-14,
and comprising an additional applicator cap as a housing part, which can be mounted at said remaining housing (13-13"") instead of said housing part (7-10, 11-11"", 12) comprising said coupling (7, 8, 9, 10) by a mechanical connection corresponding to said mechanical connection (15-24), said applicator cap being adapted for, in the state of being mounted, letting the proximal applicator part (2) or an applicator inserted instead of the latter protrude through said applicator cap in said stroke direction outwards for contacting said body to be treated.

## Revendications

1. Appareil pour le traitement du corps humain ou animal, comportant:
- un applicateur (2, 5) destiné à une application sur ledit corps depuis l'extérieur,
- un boîtier (9, 11-11"", 12) destiné à retenir l'applicateur (2, 5), et
- un mécanisme (1) permettant à l'applicateur (2, 5) d'exercer des coups par rapport au boîtier (9, 11-11"", 12) dans une direction d'impact, de manière que les coups peuvent être répercutés dans le corps suite à ladite application,
ledit applicateur (2, 5) étant retenu dans le boîtier (9, 11-11"", 12) dans un coupleur (7, 8, 9, 10),
au moins une partie distale d'applicateur (5) pouvant, après desserrage du coupleur (7, 8, 9, 10), être dégagée vers l'avant hors du coupleur (7, 8, 9, 10) et du boîtier (9, 11-11"", 12) sans devoir démonter une autre partie du boîtier, et
ledit coupleur (7, 8, 9, 10) pouvant être desserré par déplacement d'une douille de blocage (8), conçue pour retenir l'applicateur (2, 5) sous l'effet d'au moins un élément à complémentarité de forme (7) sollicité vers l'intérieur, sur l'applicateur (2, 5), dans une direction perpendiculaire à la direction d'impact, et conçue pour le libérer en permettant un mouvement de retour de l'élément à complémentarité de forme (7),
**caractérisé en ce que** l'au moins un élément à complémentarité de forme (7) rentre dans une rainure côté applicateur qui laisse de la place à l'élément à complémentarité de forme (7) pour un mouvement d'impact de l'applicateur (2, 5) vers l'avant.

2. Appareil pour le traitement du corps humain ou animal, comportant:
- un applicateur (2, 5) destiné à une application sur ledit corps depuis l'extérieur,
- un boîtier (9, 11-11"", 12) destiné à retenir l'applicateur (2, 5), et
- un mécanisme (1) permettant à l'applicateur (2, 5) d'exercer des coups par rapport au boîtier (9, 11-11"", 12) dans une direction d'impact, de manière que les coups peuvent être répercutés dans le corps suite à ladite application,
ledit applicateur (2, 5) étant retenu dans le boîtier (9, 11-11"", 12) dans un coupleur (7, 8, 9, 10),
au moins une partie distale d'applicateur (5) pouvant, après desserrage du coupleur (7, 8, 9, 10), être dégagée vers l'avant hors du coupleur (7, 8, 9, 10) et du boîtier (9, 11-11"", 12) sans devoir démonter une autre partie du boîtier, et
ledit applicateur (2, 5) se composant d'au moins deux parties, une partie distale d'applicateur (5) pouvant être dégagée après desserrage du coupleur (7, 8, 9, 10) et une partie proximale d'applicateur (2) demeurant dans le boîtier, la partie proximale d'applicateur (2) transmettant l'impact sur la partie distale d'applicateur (5),
**caractérisé en ce que** le coupleur (7, 8, 9, 10) est prévu dans une partie (7-10, 11-11"", 12) du boîtier qui est tournée vers le corps à traiter, laquelle partie de boîtier (7-10, 11-11"", 12) peut être ôtée du reste du boîtier (13-13"") par desserrage d'un raccord mécanique (15-24) conjointement au coupleur (7, 8, 9, 10), et
**caractérisé par** un capuchon d'applicateur, comme partie de boîtier, qui est susceptible d'être fixé sur le reste du boîtier (13-13"") à la place de la partie de boîtier (7-10, 11-11"", 12) présentant le coupleur (7, 8, 9, 10), par le biais d'un raccord mécanique correspondant au raccord mécanique (15-24), et qui est conçu, dans cet état de fixation, pour amener la partie proximale d'applicateur (2), ou un applicateur installé à la place de cette dernière, à faire saillie vers l'extérieur dans la direction d'impact en traversant le capuchon d'applicateur et à venir en contact avec ledit corps à traiter.

3. Appareil selon la revendication 1 ou 2, dans lequel le mécanisme (1) permettant d'exercer des coups présente un projectile et un dispositif (1) permettant une accélération du projectile de telle manière que le projectile frappe l'applicateur (2, 5) en produisant ainsi ledit coup, s'agissant de préférence d'un dispositif pneumatique d'accélération du projectile.

4. Appareil selon l'une quelconque des revendications précédentes, dans lequel l'applicateur (2, 5) est retenu dans l'appareil par complémentarité de forme sans possibilité de rotation, plus précisément sans possibilité de rotation sur un axe de rotation parallèle à la direction d'impact, une goupille polygonale (5a) entrant dans la réalisation de la complémentarité de forme de préférence du côté applicateur, en s'étendant dans la direction d'impact et, de l'autre côté, un évidement (6) qui y est adapté.

5. Appareil selon la revendication 2, facultativement en conjonction avec la revendication 3 ou 4, dans lequel le coupleur (7, 8, 9, 10) peut être desserré par déplacement d'une douille de blocage (8), conçue pour retenir l'applicateur (2, 5) sous l'effet d'au moins un élément à complémentarité de forme (7) sollicité vers l'intérieur, sur l'applicateur (2, 5), dans une direction perpendiculaire à la direction d'impact, et conçue pour le libérer en permettant un mouvement de retour de l'élément à complémentarité de forme (7).

6. Appareil selon la revendication 5, dans lequel le coupleur (7, 8, 9, 10), en vue du desserrage, peut être manipulé par déplacement d'une partie (9) du boîtier de l'appareil contre une force de ressort, à savoir de préférence à l'encontre de la direction d'impact, et la partie de boîtier (9) déplace ou présente la douille de blocage (8), et la douille de blocage (8) présente de préférence des surfaces inclinées destinées à solliciter l'élément à complémentarité de forme, et dans lequel l'élément à complémentarité de forme (7) comprend de préférence au moins deux billes.

7. Appareil selon les revendications 2 et 6, facultativement également en conjonction avec la revendication 3, 4 ou 5, dans lequel l'au moins un élément à complémentarité de forme (7) rentre dans une rainure côté applicateur qui laisse de la place à l'élément à complémentarité de forme (7) pour un mouvement d'impact de l'applicateur (2, 5) vers l'avant.

8. Appareil selon la revendication 1, facultativement en conjonction avec l'une des revendications 3 à 7, dans lequel l'applicateur (2, 5) se compose d'au moins deux parties, une partie distale d'applicateur (5) pouvant être dégagée après desserrage du coupleur (7, 8, 9, 10) et une partie proximale d'applicateur (2) demeurant dans le boîtier, la partie proximale d'applicateur (2) transmettant l'impact sur la partie distale d'applicateur (5), en sachant que, de préférence, la partie proximale d'applicateur (2) repose, avant la production d'un impact, contre un élément élastique (3) situé entre cette partie proximale d'applicateur (2) et une autre partie (4) de l'appareil qui limite le mouvement d'impact de la partie proximale d'applicateur (2) et ramène cette dernière dans sa position d'origine.

9. Appareil selon l'une quelconque des revendications précédentes, dans lequel le coupleur (7, 8, 9, 10) est prévu dans une partie (7-10, 11', 12) du boîtier tournée vers le corps à traiter, laquelle partie de boîtier (7-10, 11', 12) peut être ôtée du reste du boîtier (13') par desserrage d'un raccord à baïonnette (15, 16) conjointement au coupleur (7, 8, 9, 10).

10. Appareil selon l'une quelconque des revendications 1 à 8, dans lequel le coupleur (7, 8, 9, 10) est prévu dans une partie (7-10, 11", 12) du boîtier (9, 11, 12) qui est tournée vers le corps à traiter, laquelle partie de boîtier (7-10, 11", 12) peut être ôtée du reste du boîtier (13") par desserrage d'un raccord à anneau de serrage (17-20) conjointement au coupleur (7, 8, 9, 10), ledit raccord à anneau de serrage (17-20) présentant un anneau de serrage (17-20) qui entoure une zone de chevauchement entre la partie de boîtier (7-10, 11", 12) et le reste du boîtier (13").

11. Appareil selon la revendication 10, dans lequel l'anneau de serrage (17-20) peut être serré au moyen d'un levier excentrique (19).

12. Appareil selon l'une quelconque des revendications 1 à 8, dans lequel le coupleur (7, 8, 9, 10) est prévu dans une partie (7-10, 11‴, 12) du boîtier qui est tournée vers le corps à traiter, laquelle partie de boîtier (7-10, 11‴, 12) peut être ôtée du reste du boîtier (13'") par desserrage d'un raccord à goupille de verrouillage (21) conjointement au coupleur (7, 8, 9, 10), ledit raccord à goupille de verrouillage (21) présentant au moins une et de préférence deux goupilles de verrouillage (21) mobiles de manière perpendiculaire par rapport à la direction d'impact et conçues pour produire un verrouillage par complémentarité de forme dans une zone de chevauchement entre la partie de boîtier (7-10, 11‴, 12) et le reste du boîtier (13'").

13. Appareil selon l'une quelconque des revendications 1 à 8, dans lequel le coupleur (7, 8, 9, 10) est prévu dans une partie (7-10, 11"", 12) du boîtier qui est tournée vers le corps à traiter, laquelle partie de boîtier (7-10, 11"", 12) peut être ôtée du reste du boîtier (13"") par desserrage d'un raccord à complémentarité de forme (22-24) conjointement au coupleur (7, 8, 9, 10), le raccord à complémentarité de forme (22-24) présentant au moins une bille (22) comme élément à complémentarité de forme, laquelle est susceptible d'être mise en mouvement perpendiculairement à la direction d'impact du fait du mouvement d'une douille de manipulation (24) dans une zone de chevauchement de la partie de boîtier (7-10, 11"", 12) et du reste du boîtier (13ʺʺ) en produisant ainsi une complémentarité de forme établie et suspendue par la coopération entre la bille (22) et un évidement adapté.

14. Appareil selon la revendication 13, dans lequel la douille de manipulation (24) est mobile dans un sens circonférentiel autour de la direction d'impact et présente, sur une face intérieure, un évidement permettant le mouvement de l'au au moins une bille (22).

15. Appareil selon les revendications 1 et 8, dans lequel le coupleur (7, 8, 9, 10) est prévu dans une partie (7-10, 11-11"", 12) du boîtier tournée vers le corps à traiter, laquelle partie de boîtier (7-10, 11-11"", 12) peut être ôtée du reste du boîtier (13-13"") par desserrage d'un raccord mécanique (15-24) conjointement au coupleur (7, 8, 9, 10), ledit raccord mécanique (15-24) correspondant de préférence à l'une des revendications 10 à 14,
et comportant encore, comme partie de boîtier, un capuchon d'applicateur qui est susceptible d'être fixé sur le reste du boîtier (13-13"") à la place de la partie de boîtier (7-10, 11-11"", 12) présentant le coupleur (7, 8, 9, 10), par le biais d'un raccord mécanique correspondant au raccord mécanique (15-24), et qui est conçu, dans cet état de fixation, pour amener la partie proximale d'applicateur (2), ou un applicateur installé à la place de cette dernière, à faire saillie vers l'extérieur dans la direction d'impact en traversant le capuchon d'applicateur et à venir en contact avec ledit corps à traiter.
